# EUROPEAN PATENT SPECIFICATION

(11) **EP 2 583 566 B1**
(45) Date of publication and mention of the grant of the patent: **19.08.2015**
(21) Application number: 11186150.6
(22) Date of filing: 21.10.2011
(51) Int. Cl.: A23L 1/305, A61K 35/20, A23L 1/29, A61P 21/06

(54) **Whey protein micelles to enhance muscle mass and performance**
Molkeproteinmizellen zur Verbesserung der Muskelmasse und -leistung
Micelles de protéine de lactosérum pour améliorer la masse musculaire et la performance

(43) Date of publication of application: 24.04.2013
(73) Proprietor: Nestec S.A., 1800 Vevey (CH)
(72) Inventor: BREUILLE, Denis, 1010 LAUSANNE (CH); MOORE, Daniel, 1000 LAUSANNE (CH); STELLINGWERFF, Trent, 1052 LE MONT-SUR-LAUSANNE (CH); POUTEAU, Etienne, 7550042 SANTIAGO (CL); BOVETTO, Lionel, 74500 LARRINGES (FR)
(74) Representative: Rupp, Christian

(56) References cited:
- WO-A1-2011/011252
- WO-A1-2011/112695
- WO-A2-2006/034857
- US-A- 5 882 705
- US-A1- 2011 250 310
- ACHESON K J ET AL: "Protein choices targeting thermogenesis and metabolism", THE AMERICAN JOURNAL OF CLINICAL NUTRITION, AMERICAN SOCIETY FOR NUTRITION, US, vol. 93, no. 3, 1 January 2011 (2011-01-01), pages 525-534, XP009156726, ISSN: 0002-9165

## Description

The present invention relates to a non-therapeutic use of whey protein micelles to enhance muscle protein synthesis in a subject. Further aspects of the invention are food compositions comprising whey protein micelles to be administered to children, athletes or elderly persons.

Whey protein is an excellent protein choice for individuals of all ages who value the role of a healthy diet in helping to maintain and improve their health, strength and physical performance. Whey protein isolate, the purest form available, is still currently unsurpassed as a source of the essential amino acids required in a daily diet. Essential amino acids are the building blocks for healthy muscles, skin, nails and other body tissues.

Whey protein has long been considered the 'gold standard' of protein for serious athletes who work hard to develop and sustain a lean, strong and well defined physique. Athletes need more protein in their diet, often as much as twice the recommended daily allowance. The protein they choose makes a difference and there are several reasons why whey protein is a preferred choice for athletes of all types. Whey protein is a naturally complete protein, containing all the essential amino acids required in an ideal combination to help improve body composition and to enhance athletic performance. Whey protein is a rich source of branched chain amino acids (BCAAs). This is important for athletes, since those BCAAs are metabolized directly into muscle tissue and are the first amino acids used during periods of exercise and resistance training. Whey protein is also an excellent source of the amino acid leucine. Leucine is important for athletes as it plays a key role in promoting muscle protein synthesis and muscle growth.

Dietary protein is very important for growth of infants and children. Whey protein contains many of the same components as can be found in human breast milk and for this reason is a key ingredient in many infant formulas. Thereby, the natural composition of the whey protein as protein source rich in essential amino acids, BCAAs and leucine, supports the healthy growth and build-up of muscle tissue for the growing up child, in a very similar way as it does for athletes.

As we age, muscle loss and its negative health implications is a growing concern. Good nutrition and adequate amounts of high quality whey protein can help to maintain strong muscles during aging, especially when combined with physical exercise and resistance training. A recent study found that older men who consumed whey protein showed greater protein synthesis and muscle growth, which helped to limit loss of muscle over time.

Tang JE et al. (2009, J Appl Physiol 107:987-992) investigated the response of skeletal muscle protein synthesis in young men following the ingestion of three distinct but high-quality dietary proteins, i.e. whey, micellar casein and soy, at rest and after resistance exercise. Thereby, the authors reported that the consumption of whey proteins stimulated muscle protein synthesis to a greater degree than casein, both at rest and after resistance exercise. Whey proteins stimulated also a significantly larger rise in muscle synthesis than soy proteins, which was in congruence with previous work of the same authors. They concluded that whey proteins stimulate skeletal muscle protein synthesis to a greater extent than either casein or soy proteins, both at rest and after resistance exercise.

There is still a persisting need in the food industry to find even better nutritional solutions than whey protein isolate to enhance muscle mass, strength and performance for healthy individuals of all age.

The use of whey protein micelles to support muscle mass has been addressed in e.g., WO 2011/112695, US 2011/250310 and WO 2011/011252.

The object of the present invention is to improve the state of the art and to provide a nutritional solution to enhance muscle protein synthesis in a healthy subject.

The object of the present invention is achieved by the subject matter of the independent claim. The dependent claims further develop the idea of the present invention.

Accordingly, the present invention provides in a first aspect a non-therapeutic use of whey protein micelles to enhance muscle protein synthesis in a subject by inducing a delayed hyper-aminoacidemia in the subject, wherein the whey protein micelles are provided to the subject in a daily dose of at least 20 g dry weight, and wherein the whey protein micelles are provided in combination with a meal, and wherein the meal comprises whey protein isolates, native or hydrolysed milk proteins, free amino acids or a combination thereof, wherein the combination of the whey protein micelles and the meal comprises 15-50% proteins, 10-15% lipids, 25-50% carbohydrates and 5-10% fibers of total dry weight.

In a second aspect, the invention relates to a food composition comprising whey protein micelles, wherein the food composition is to be administered to an athlete, an elderly person, an infant or a child.

"Whey protein micelles" (WPM) are defined herein as described in EP1839492A1 and as further characterized in Schmitt C et al. (2010, Soft Matter 6:4876-4884), where they are referred to as whey protein microgels (WPM). Particularly, the "whey protein micelles" are the micelles comprised in the whey protein micelles concentrate obtainable by the process as disclosed in EP1839492A1. Therein, the process for the production of whey protein micelles concentrate comprises the steps of: a) adjusting the pH of a whey protein aqueous solution to a value between 3.0 and 8.0; b) subjecting the aqueous solution to a temperature between 80 and 98°C; and c) concentrating the dispersion obtained in step b). Thereby, the micelles produced have an extremely sharp size distribution, such that more than 80% of the micelles produced have a size smaller than 1 micron in diameter and preferably are between 100 nm and 900 nm in size. The "whey protein micelles" can be in liquid concentrate or in powder form. Importantly, the basic micelle structure of the whey proteins is conserved, in the concentrate, the powder and reconstituted from the powder for example in water. The "whey protein micelles" are physically stable in dispersion, as powder as well as during spray-drying or freeze-drying.

A rapid increase in plasma amino acids is required for stimulating muscle protein synthesis at rest and after exercise (Dangin M et al., 2003, J Physiol 549:635-644). One of the currently best solutions for providing this rapid increase in plasma amino acids is whey protein isolate (WPI) (Tang JE et al., 2009, J Appl Physiol 107:987-992). A more sustained amino acid response may prolong the increase in anabolism and muscle protein synthesis by providing amino acid building blocks over a longer period of time (Lacroix M et al., 2006, Am J Clin Nutr 84:1070-9). In addition, a more slowly digested protein may suppress protein breakdown (Dangin M et al., 2001, Am J Physiol 280:E340-E348), which would have an additional benefit for the net muscle protein balance, i.e. the difference between protein synthesis and protein breakdown. Thus, a protein or a mix of proteins that would induce the maximal aminoacidemia but during a longer period of time would do both, i.e. maximally stimulate protein synthesis and suppress protein breakdown.

It has now been surprisingly found by the inventors that whey protein micelles consumed as part of a meal induce the same high plasma aminoacidemia as an iso-caloric and iso-nitrogenous control meal with whey protein isolates (WPI), but significantly delayed postprandially by about 30 min with respect to that of the control meal. The peak amino acid concentration (i.e. Cmax) after the whey protein micelles meal was the same as after the WPI meal, and significantly higher than the maximum concentrations reached after an iso-caloric and iso-nitrogenous milk protein or milk casein meal. The results of the clinical study are presented in the Example section.

Hence, the inventors have found a protein composition which when consumed as part of a regular meal induces a delayed but high maximal aminoacidemia in a subject. This hyper-aminoacidemia for a prolonged postprandial period of time is most favourable for maximally stimulating muscle protein synthesis, decreasing muscle protein breakdown and therefore maintaining and/or enhancing muscle mass.

"Hyper-aminoacidemia" is an excess of amino acids in the bloodstream, the amino acid pool, which can lead to an increase in protein synthesis and reduction of protein breakdown, with an overall positive nitrogen balance. Thereby, the positive nitrogen balance indicates more construction of lean tissue than destruction, leading overall to an increase in lean body mass.

Although not wishing to be bound by theory, the inventors think that whey protein micelles as part of a meal seem to induce a delayed gastric emptying and to be more slowly digested as compared to native whey proteins such as WPI. Thereby, whey protein micelles deliver the amino acids more slowly into the peripheral blood circulation.
Figure 1: Plasma concentrations of essential amino acids 3 h after the ingestion of meal replacements comprising whey protein isolate, whey protein micelles or micellar casein.
Figure 2: Plasma concentrations of leucine 3 h after the ingestion of meal replacements comprising whey protein isolate, whey protein micelles or micellar casein.
Figure 3: Plasma concentrations of essential amino acids 3 h after the ingestion of meal replacements comprising each one of the 7 different proteins.

The present invention pertains to a non-therapeutic use of whey protein micelles to enhance muscle protein synthesis in a subject and thereby to increase muscle mass, muscle strength and/or muscle performance. The hyper-aminoacidemia for a prolonged postprandial period of time provided by the inventive use of the whey protein micelles is most favourable for maximally stimulating muscle protein synthesis and therefore maintaining and/or enhancing muscle mass, resulting in increased muscle strength and performance.

The non-therapeutic use according to the invention is for a human being, preferably an infant, a growing-up child, an athlete or an elderly person.

"Infant" means a child under the age of 36 months.

"Athlete" means a person possessing the natural or acquired traits, such as strength, agility and endurance that are necessary for physical exercise or sports, especially those performed in competitive contexts.

"Elderly person" means a person with a chronological age of 65 years or older.

Infants, children who are still in the growing-up phase, athletes and elderly persons have in common the need of elevated muscle protein synthesis: infants for their rapid growth and build up of muscle tissue, athletes for further building up muscle mass and muscle performance, and elderly persons for at least maintaining and compensating the natural loss of muscle mass due to aging. Hence it is those persons who will best gain from the current invention.

The non-therapeutic use of whey protein micelles according to the invention pertains also to an animal, preferably a cat or a dog. Owners of animals, particularly those animals kept as pet animals like cats and dogs, may wish to increase the muscle mass of his pet animal to increase the muscle force and performance for example for racing or other competitive purposes. Alternatively, the non-therapeutic use of whey protein micelles according to the invention pertains also to horses and cattle.

The whey protein micelles are provided to a subject in a daily dose of at least 20 g dry weight, preferably of at least 30 g dry weight. Those doses should assure a sufficient daily quantity for providing the desired effect to a subject in at least a mid-term period.

The whey protein micelles are provided in combination with a meal. Most meals comprise proteins from a milk, plant and/or animal source and hence upon consumption lead to a postprandial increase in aminoacidemia, i.e. an elevated concentration of amino acids in the plasma of the consumer. It is now an advantage, to combine the administration of whey protein micelles in combination with such a meal. Thereby, the postprandial plasma amino acid peak resulting from the proteins present in the meal adds up to the postprandial amino acid peak resulting from the whey protein micelles which are delayed by ca. 30 min in respect to the first amino acid peak. Thereby, the overall resulting hyper-aminoacidemia is extended and prolonged in time. This in return is most favourable for maximally stimulating muscle protein synthesis and therefore maintaining or enhancing muscle mass.

The meal comprises whey protein isolates, native or hydrolyzed milk proteins, free amino acids, or a combination thereof. As known from earlier studies, a whey protein meal exhibits a significantly stronger aminoacidemia effect on subjects than for example a plant protein meal. Therefore, advantageously, the whey protein micelles are combined with a meal comprising whey proteins in the form of WPI or milk. Advantageously, the meal can be even further supplemented with free amino acids in combination with the whey or milk proteins to optimally induce a hyper-aminoacidemia upon consumption of said meal.

The combination of the whey protein micelles and the meal comprises 15-50 wt% proteins, 10-15 wt% lipids, 25-50 wt% carbohydrates and 5-10 wt% fiber of the total dry weight of the combination.

In a preferred embodiment, the whey protein micelles are provided together and/or as part of a meal in the form of a beverage, nutritional composition, bar, flakes, biscuits or as pellets. Thereby, the different individual protein components can be optimally dosed for providing a best and prolonged hyper-aminoacidemia effect and at the same time optimized for a good, organoleptically best product application. Furthermore, it is convenient for a consumer to have the whey protein micelles with the meal in one single consumable food product, like for example a beverage or bar.

The present disclosure also relates to a food composition comprising whey protein micelles, wherein the food composition is to be administered to an athlete or an elderly person. The food composition can for example be in the form of a bodybuilding supplement, a sports nutrition bar, a nutrition sports beverage, or a food supplement for seniors.

The present disclosure also relates to a food composition for an infant or a child. The food composition can for example be an infant feeding formula, a milk drink or shake, a fermented or acidified milk product in the form e.g. of a yoghurt or dessert, a biscuit, an ice cream.

The present disclosure also relates to a food composition for animals comprising whey protein micelles. Preferably, the food composition is for a cat, a dog, a horse or cattle.

Further advantages and features of the present invention are apparent from the figures and examples.

### Example

A randomized double-blind 7-arm crossover study was performed in twenty-three healthy men in the following way. A test meal replacement was ingested at lunch time on 7 separate occasions separated each by a wash-out period of one week. The meal replacements were iso-caloric and iso-nitrogenous. They were composed of the tested protein (30g, 7.2% w/w), lipids (11.7g, 2.8% w/w), carbohydrates (42.7g, 10.2% w/w) and fibers (6.3g, 1.5% w/w). The tested proteins were: (1) whey protein isolate (WPI); (2) whey protein micelles (WPM); (3) extensively hydrolyzed whey protein (EHWP); (4) micellar casein (ICP); (5) extensively hydrolyzed casein protein (EHCP); (6) total milk proteins (TMP); and (7) extensively hydrolyzed milk proteins (EHMP). The meal replacements were completed with water to 430mL and contained 388kcal per serving.

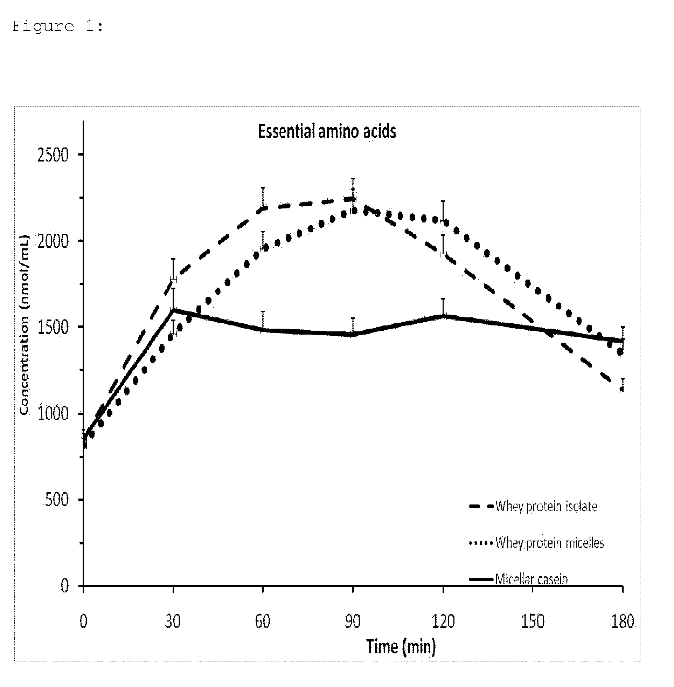

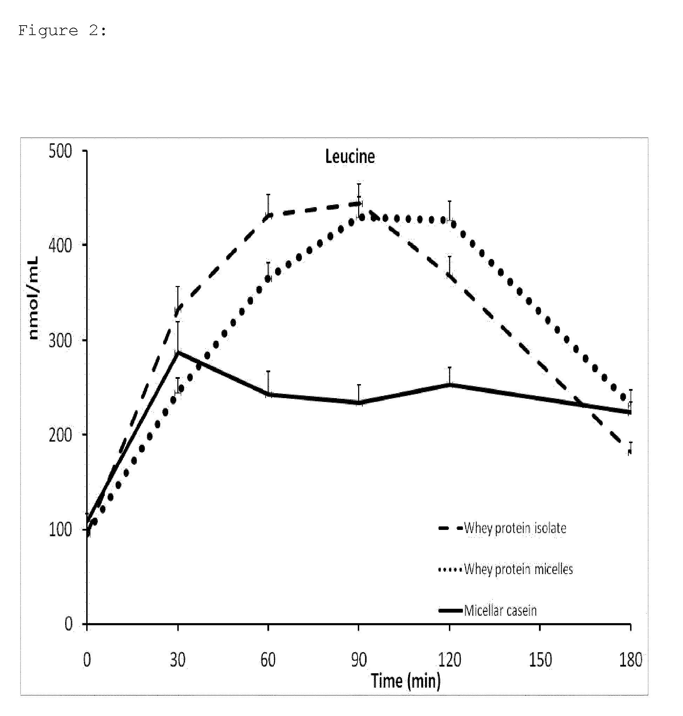

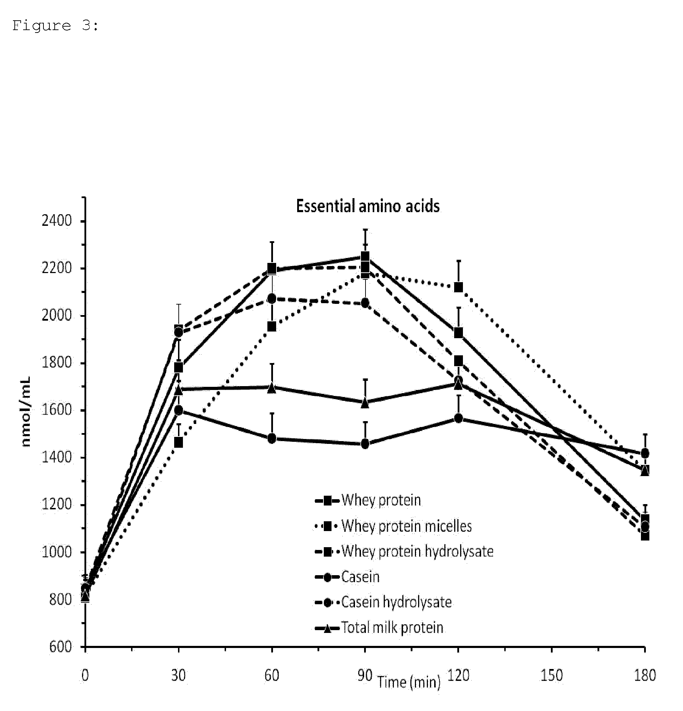

Arterialized venous blood samples were taken, via a catheter inserted into a wrist vein of the volunteers, before and for 3h after consuming the test meal replacement. Plasma samples were used to analyze amino acids by gas chromatography and mass spectrometry. The results are shown in Figures 1 to 3. Firstly, the results confirmed that intact whey protein induces a higher aminoacidemia than micellar casein. Secondly, it was found that the peaks of the postprandial plasma amino acid concentrations after consumption of the WPI and WPM test meal replacements, although similar in extent and height, were delayed by approximately 30 min, i.e. occurring at 120 min rather than at 90 min. This allowed maintenance of an elevated concentration of plasma amino acids for a prolonged period of time after the ingestion of the whey protein micelles (Figures 1 to 3: small dotted lines).

## Claims

1. Non-therapeutic use of whey protein micelles wherein the whey protein micelles are provided to a subject in a daily dose of at least 20 g dry weight, and wherein the whey protein micelles are provided in combination with a meal, and wherein the meal comprises whey protein isolates, native or hydrolyzed milk proteins, free amino acids or a combination thereof, wherein the combination of the whey protein micelles and the meal comprises 15-50 wt% proteins, 10-15 wt% lipids, 25-50 wt% carbohydrates and 5-10 wt% fibers of total dry weight, to enhance muscle protein synthesis in a subject by inducing a delayed hyper-aminoacidemia in the subject.

2. The non-therapeutic use according to claim 1, to increase muscle mass, muscle strength and/or muscle performance.

3. The non-therapeutic use according to one of the preceding claims, wherein the subject is an infant, a growing-up child, an athlete or an elderly person.

4. The non-therapeutic use according to one of the claims 1-2, wherein the subject is an animal, preferably a cat or a dog.

5. The non-therapeutic use according to one of the preceding claims, wherein the whey protein micelles are provided to the subject in a daily dose of at least 30 g dry weight.

6. The non-therapeutic use according to one of the claims 1 to 5, wherein the whey protein micelles are provided as part of the meal in the form of a beverage, nutritional composition, bar, flakes, biscuits, or as pellets.

## Patentansprüche

1. Nicht-therapeutische Verwendung von Molkeproteinmizellen, wobei die Molkeproteinmizellen einem Probanden in einer täglichen Dosis von mindestens 20 g Trockengewicht gegeben werden, und wobei die Molkeproteinmizellen in Kombination mit einer Mahlzeit gegeben werden, und wobei die Mahlzeit Molkeproteinisolate, native oder hydrolysierte Milchproteine, freie Aminosäuren oder eine Kombination davon aufweist, wobei die Kombination der Molkeproteinmizellen und der Mahlzeit 15-50 Gew.-% Proteine, 10-15 Gew.-% Lipide, 25-50 Gew.-% Kohlenhydrate und 5-10 Gew.-% Fasern des Gesamttrockengewichts aufweist, um die Muskelproteinsynthese bei einem Probanden durch Herbeiführen einer verzögerten Hyperaminoazidämie bei dem Probanden zu verbessern.

2. Nicht-therapeutische Verwendung nach Anspruch 1, um die Muskelmasse, die Muskelkraft und/oder die Muskelleistung zu erhöhen.

3. Nicht-therapeutische Verwendung nach einem der vorhergehenden Ansprüche, wobei der Proband ein Säugling, ein heranwachsendes Kind, ein Sportler oder ein älterer Mensch ist.

4. Nicht-therapeutische Verwendung nach einem der Ansprüche 1-2, wobei der Proband ein Tier, vorzugsweise eine Katze oder ein Hund, ist.

5. Nicht-therapeutische Verwendung nach einem der vorhergehenden Ansprüche, wobei die Molkeproteinmizellen dem Probanden in einer täglichen Dosis von mindestens 30 g Trockengewicht gegeben werden.

6. Nicht-therapeutische Verwendung nach einem der Ansprüche 1 bis 5, wobei die Molkeproteinmizellen als Teil einer Mahlzeit in Form eines Getränks, einer Nahrungszusammensetzung, eines Riegels, Flocken, Keksen oder als Pellets gegeben werden.

## Revendications

1. Utilisation non thérapeutique de micelles de protéine de lactosérum, dans laquelle les micelles de protéine de lactosérum sont fournies à un sujet en une dose quotidienne d'au moins 20 g de poids sec, et dans laquelle les micelles de protéine de lactosérum sont fournies en combinaison avec un repas, et dans laquelle le repas comprend des isolats de protéine de lactosérum, des protéines de lait natives ou hydrolysées, des acides aminés libres ou une combinaison de ceux-ci, dans laquelle la combinaison des micelles de protéine de lactosérum et du repas comprend 15-50 % en poids de protéines, 10-15 % en poids de lipides, 25-50 % en poids de glucides et 5-10 % en poids de fibres du poids sec total, afin d'améliorer la synthèse de protéines musculaires chez un sujet en provoquant chez le sujet une hyper-aminoacidemia retardée.

2. Utilisation non thérapeutique selon la revendication 1, afin d'augmenter la masse musculaire, la force musculaire et/ou la performance musculaire.

3. Utilisation non thérapeutique selon l'une des revendications précédentes, dans laquelle le sujet est un nourrisson, un enfant en croissance, un athlète ou une personne âgée.

4. Utilisation non thérapeutique selon l'une des revendications 1-2, dans laquelle le sujet est un animal, de préférence un chat ou un chien.

5. Utilisation non thérapeutique selon l'une des revendications précédentes, dans laquelle les micelles de protéine de lactosérum sont fournies au sujet en une dose quotidienne d'au moins 30 g de poids sec.

6. Utilisation non thérapeutique selon l'une des revendications à à 5, dans laquelle les micelles de protéine de lactosérum sont fournies dans le cadre du repas sous la forme d'une boisson, d'une composition nutritionnelle, d'une barre, de flocons, de biscuits, ou en tant que pellets.
